# EUROPEAN PATENT APPLICATION

(11) **EP 3 144 297 A1**
(43) Date of publication of application: **22.03.2017**
(21) Application number: 15792094.3
(22) Date of filing: 12.05.2015
(51) Int. Cl.: C07D 239/26, A01N 43/54, A01P 3/00, C07D 239/28, C07D 239/42

(54) **ETHEREAL OXYGEN ATOM-CONTAINING PERFLUOROALKYL GROUP-SUBSTITUTED PYRIMIDINE RING COMPOUND, AND METHOD FOR PRODUCING SAME**

(30) Priority: 14.05.2014 JP 2014100655
(71) Applicant: Asahi Glass Company, Limited, Tokyo 100-8405 (JP)
(72) Inventor: MORIZAWA, Yoshitomi, Tokyo 100-8405 (JP); TAKAHIRA, Yusuke, Tokyo 100-8405 (JP); JYOUMUTA, Daisuke, Tokyo 100-8405 (JP); NAKANO, Takashi, Tokyo 100-8405 (JP)
(74) Representative: Müller-Boré & Partner Patentanwälte PartG mbB
(86) International application number: PCT/JP2015/063666
(87) International publication number: WO 2015/174421

(57) **Abstract**

To provide a pyrimidine derivative having a fluorinated substituent group, that can be applied to an agricultural chemical or pharmaceutical having excellent pharmacological activities, and a method for producing said derivative. A compound represented by the following formula (A). R^{f} is a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20. R¹ is a C₁₋₆ alkyl group, etc. Q¹ is R²C(O)-, R³OC(O)-, a carboxy group, R⁴OC(O)NH-, an amino group, or R⁵C(O)NH- (R² to R⁵ are each independently a C₁₋₆ alkyl group, etc.).

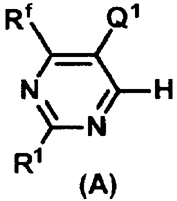

## Description

### TECHNICAL FIELD

The present invention relates to a pyrimidine ring compound having an etheric (ethereal) oxygen atom-containing perfluoroalkyl group substituted thereon, and a process for producing such a compound.

### BACKGROUND ART

Compounds having heterocyclic rings (hereinafter referred to as "heterocyclic compounds") are often found in natural products or biological components and thus are widely used as pharmacologically active substances for pharmaceuticals, agricultural chemicals, etc. Further, they are widely used also as functional materials for liquid crystal materials, organic semiconductor materials, etc. In particular, by various combinations of the types of heterocyclic rings, the number and positions of substituents, the presence or absence of aromaticity, etc., it is possible to construct a variety of structures. Among them, in recent years, utilizing unique properties possessed by fluorine atoms, many heterocyclic compounds having fluorinated substituents are put in practical use as pharmaceuticals or agricultural chemicals having excellent pharmacological activities.

In the pharmaceuticals or agricultural chemicals, most of the fluorinated substituents which heterocyclic compounds had, were fluorine atoms or perfluoroalkyl groups. In recent years, as agricultural chemicals having, as perfluoroalkyl groups, a trifluoromethyl group and a heptafluoro-2-propyl group, 4-heptafluoroisopropyl-2-(trifluoromethylthio) aniline, flubendiamide, etc. have been reported (Patent Document 1 and Non-Patent Document 1).

Among heterocyclic compounds, as pyrimidine derivatives, 2-perfluoro(2-methoxy(ethoxy) methyl)-4,6-bis-trifluoromethyl-5-fluoropyrimidine represented by the following formula (a) (Non-Patent Document 2), 2,6-diphenyl-4-trifluoromethylpyrimidine represented by the following formula (b) (Non-Patent Document 3), and 4-heptafluoropropyl-2-oxo-1,2-dihydropyrimidine-5-carboxylate represented by the following formula (c) (Non-Patent Document 4), are, for example, known.

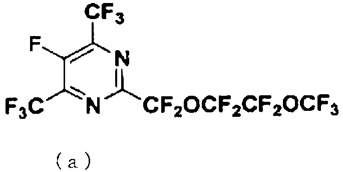

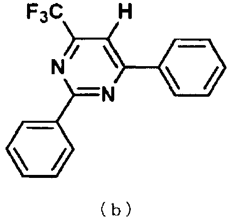

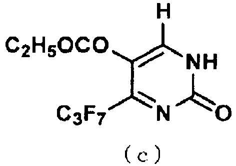

### PRIOR ART DOCUMENTS

### PATENT DOCUMENT

Patent Document 1: WO2006/137395

### NON-PATENT DOCUMENTS

Non-Patent Document 1: Fine Chemicals, Vol. 36, No. 8, pp. 58-65 (2007)
Non-Patent Document 2: Fluorine Notes (2009), p. 65
Non-Patent Document 3: Synlett, (1999), pp. 756-758
Non-patent Document 4: Russian Chemical Bulletin, International Edition, (2009) 58 (6), pp. 1,259-1,263

### DISCLOSURE OF INVENTION

### TECHNICAL PROBLEM

Some compounds have been known wherein a fluorinated alkyl group having an etheric (ethereal) oxygen atom is bonded to a pyrimidine skeleton, but a compound has not been known wherein a fluorinated alkyl group having an etheric (ethereal) oxygen atom (group R^{f} in the present invention), a hydrogen atom and a chemically convertible specific substituent (group Q¹ in the present invention), are bonded at specific positions in a pyrimidine skeleton.

It is an object of the present invention to provide a novel pyrimidine derivative and a method for its production.

### SOLUTION TO PROBLEM

The present inventors have found, as a novel compound, a compound wherein to three carbon atoms out of the four carbon atoms of a pyrimidine ring, a fluorinated alkyl group having an etheric (ethereal) oxygen atom, and specific substituents, are bonded, and to the remaining one carbon atom, a hydrogen atom is bonded, and have arrived at accomplishing the present invention.

That is, the present invention is as follows.
[1] A compound represented by the following formula (A): 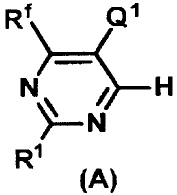 wherein R^{f}, R¹ and Q¹ have the following meanings:
   R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
   R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
   Q¹: (1) a group represented by R²C(O)- (wherein R² is a C₁₋₆ alkyl group, a phenyl group, or a group having at least one hydrogen atom bonded to a carbon atom in a C₁₋₆ alkyl group or a phenyl group, substituted each independently by a group selected from an amino group, a hydroxy group, a halogen atom, a C₁₋₆ alkoxy group, a carboxy group and a trifluoromethyl group), (2) a group represented by R³OC(O)- (wherein R³ is a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom), (3) a carboxy group, (4) a group represented by R⁴OC(O)NH- (wherein R⁴ is a C₁₋₆ alkyl group, or a group having at least one hydrogen atom bonded to a carbon atom in a C₁₋₆ alkyl group, substituted by a halogen atom), (5) an amino group, or (6) a group represented by R⁵C(O)NH- (wherein R⁵ is a C₁₋₆ alkyl group, a phenyl group, an aralkyl group, or a group having at least one hydrogen atom bonded to a carbon atom in a C₁₋₆ alkyl group, a phenyl group or an aralkyl group, substituted each independently by a group selected from an amino group, a hydroxy group, a halogen atom and a trifluoromethyl group.).
[2] The compound according to the above [1], wherein R^{f} in the formula (A) is a group represented by (R^{f10})(R^{f11})(R^{f12})C-O-(R^{f13})(R^{f14})C- (wherein R^{f10} to R^{f14} are each independently a C₁₋₁₂ perfluoroalkyl group, a C₁₋₁₂ perfluoroalkyl group having an etheric (ethereal) oxygen atom at least between carbon-carbon atoms or at a bond terminal, or a fluorine atom, and the total of the number of carbon atoms and the number of oxygen atoms is from 3 to 20.).
[3] The compound according to the above [1] or [2], wherein the compound represented by the formula (A) is a compound represented by the following formula (A1): 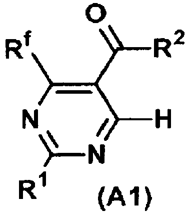 wherein R^{f}, R¹ and R² are as defined above.
[4] The compound according to the above [1] or [2], wherein the compound represented by the formula (A) is a compound represented by the following formula (A2): 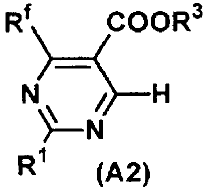 wherein R^{f}, R¹ and R³ are as defined above.
[5] The compound according to the above [1] or [2], wherein the compound represented by the formula (A) is a compound represented by the following formula (A3): 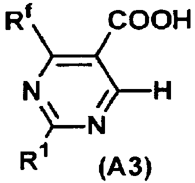 wherein R^{f} and R¹ are as defined above.
[6] The compound according to the above [1] or [2], wherein the compound represented by the formula (A) is a compound represented by the following formula (A5): 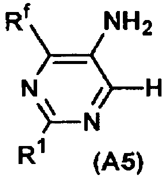 wherein R^{f} and R¹ are as defined above.
[7] The compound according to the above [1] or [2], wherein the compound represented by the formula (A) is a compound represented by the following formula (A6): 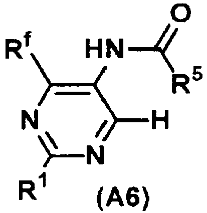 wherein R^{f}, R¹ and R⁵ are as defined above.
[8] The compound according to any one of the above [1] to [7], wherein R¹ is a phenyl group, or a group having at least one hydrogen atom bonded to a carbon atom in a phenyl group, substituted each independently by a group selected from a hydroxy group, an amino group, a carboxy group and a halogen atom.
[9] A method for producing a compound represented by the following formula (A1), which comprises reacting a compound represented by the following formula (9) and a compound represented by the following formula (5): 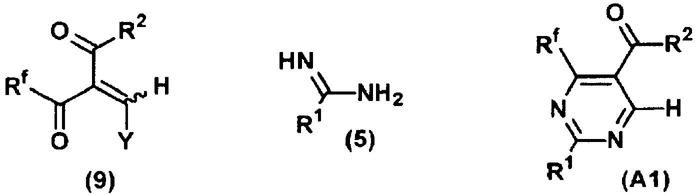 wherein
   R^{f}, Y, R¹ and R² have the following meanings:
   R^{f}: a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
   Y: a group represented by -OR' or -NR"₂, wherein R' and R" are each independently a C₁₋₃ alkyl group,
   R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
   R²: a C₁₋₆ alkyl group, a phenyl group, or a group having at least one hydrogen atom bonded to a carbon atom in a C₁₋₆ alkyl group or a phenyl group, substituted each independently by a group selected from an amino group, a hydroxy group, a halogen atom, a C₁₋₆ alkoxy group, a carboxy group and a trifluoromethyl group.
[10] A method for producing a compound represented by the following formula (A2), which comprises reacting a compound represented by the following formula (10) and a compound represented by the following formula (5): 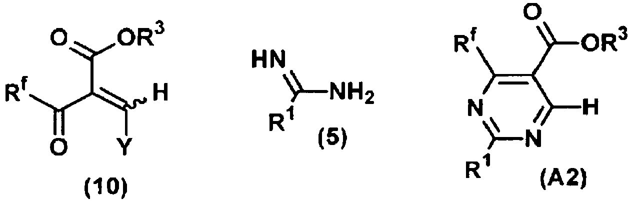 wherein
   R^{f}, Y, R¹ and R³ have the following meanings:
   R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
   Y: a group represented by -OR' or -NR"₂, wherein R' and R" are each independently a C₁₋₃ alkyl group,
   R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
   R³: a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom.
[11] A method for producing a compound represented by the following formula (A6), which comprises hydrolyzing a compound represented by the following formula (A2) under a basic condition, to obtain a compound represented by the following formula (A3); converting the compound represented by the formula (A3) to a mixed acid anhydride, and then to an azide ketone by an metal azide compound; while further letting it undergo the rearrangement reaction, reacting it with a compound represented by the formula R⁴-OH (wherein R⁴ is a C₁₋₆ alkyl group, or a group having at least one hydrogen atom bonded to a carbon atom in a C₁₋₆ alkyl group, substituted by a halogen atom) to obtain a compound represented by the following formula (A4), then reacting the compound represented by formula (A4) with an acid, to obtain a compound represented by the following formula (A5), and then reacting the compound represented by the formula (A5) with a compound represented by the formula R⁵COX (wherein R⁵ is a C₁₋₆ alkyl group, a phenyl group, an aralkyl group, or a group having at least one hydrogen atom bonded to a carbon atom in a C₁₋₆ alkyl group, a phenyl group or an aralkyl group, substituted each independently by a group selected from an amino group, a hydroxy group, a halogen atom and a trifluoromethyl group, and X is a halogen atom, a group obtained by removing a hydrogen atom from N-hydroxysuccinimide, a C₁₋₆ alkoxy group, a perfluorophenoxy group or a C₁₋₆ fluoroalkoxy group.). 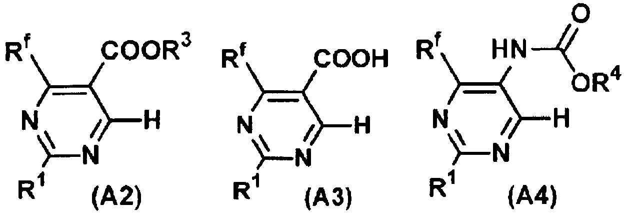 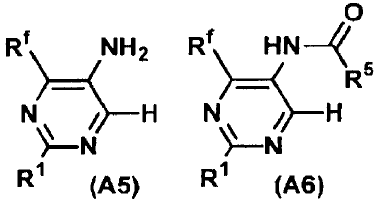 wherein
   R^{f}, R¹ and R³ have the following meanings, and R⁴ and R⁶ are as defined above:
      R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
      R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
      R³: a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom.
[12] An agricultural chemical containing a compound selected from the compound as defined in any one of the above [1] to [8] and pharmacologically effective salts of said compound.

### ADVANTAGEOUS EFFECTS OF INVENTION

The compound of the present invention is a novel pyrimidine derivative wherein to three carbon atoms out of the four carbon atoms of the pyrimidine ring, a fluorinated alkyl group having an etheric (ethereal) oxygen atom, and two specific substituents, are respectively bonded, and to the remaining one carbon atom, a hydrogen atom is bonded, and an ester bond, an amide bond or a carboxy group, as the substituent, may be changed by conversion reactions to form various pyrimidine ring compounds, and thus, it is also useful as a novel intermediate for pyrimidine ring compounds.

Further, the compound of the present invention is useful as a drug substance for new pharmaceuticals and agricultural chemicals, and may exhibit high pharmacological activities.

### DESCRIPTION OF EMBODIMENTS

The present invention will be described in detail, but is not limited to the embodiments disclosed in the following description.

In the present specification, a compound represented by the formula (n), may be expressed as a "compound (n)". A perfluoroalkyl group means a group having all of hydrogen atoms in an alkyl group substituted by fluorine atoms. An alkyl group represents a linear or branched group unless otherwise specified.

The compound of the present invention is represented by the following formula (A).

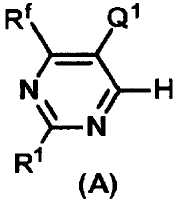

In the formula (A), R^{f} is a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20. The total of the number of carbon atoms and the number of oxygen atoms in R^{f} is preferably from 3 to 12, particularly preferably from 6 to 10. The number of carbon atoms in R^{f} is preferably from 2 to 10. The number of oxygen atoms is preferably 1 or 2. R^{f} may be linear or branched.

The compound (A) is a pyrimidine derivative wherein a perfluoroalkyl group (R^{f}) having an etheric (ethereal) oxygen is bonded to a carbon atom at a specific position on the pyrimidine ring. The group represented by R^{f} has an etheric (ethereal) oxygen atom and thus is a group capable of being bended, and, it has fluorine atoms and thus has hydrophobicity, whereby when used as a pharmaceutical, agricultural chemical, etc., it becomes possible to bond to an active site to express a physiological activity.

R^{f} is preferably a group represented by the following formula (23). In the following formula (23), R^{f10} to R^{f14} are each independently a C₁₋₁₂ perfluoroalkyl group, a C₁₋₁₂ perfluoroalkyl group having an etheric (ethereal) oxygen atom between carbon-carbon atoms, or a fluorine atom, and the total of the number of carbon atoms and the number of oxygen atoms is from 3 to 20.

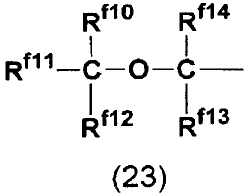

In a case where any of R^{f10} to R^{f14} is a C₁₋₁₂ perfluoroalkyl group, a C₁₋₈ group is preferred, and a trifluoromethyl group, a pentafluoroethyl group, a heptafluoro(n-propyl) group, a heptafluoroisopropyl group, a nonafluoro(n-butyl) group, a nonafluoroisobutyl group, a perfluoro(n-hexyl) group, a perfluoro(n-octyl) group, etc. are preferred.

In a case where any of R^{f10} to R^{f14} is a C₁₋₁₂ perfluoroalkyl group having an etheric (ethereal) oxygen atom, a C₁₋₈ group having from 1 to 3 etheric (ethereal) oxygen atoms is preferred, and a perfluoro(methoxymethyl) group, a perfluoro(ethoxymethyl) group, a perfluoro(isopropoxymethyl) group, a perfluoro(1-methoxyethyl) group, a perfluoro(1-ethoxyethyl) group, a perfluoro((2-methoxy)ethoxymethyl) group, a perfluoro((2-ethoxy)ethoxymethyl) group, a perfluoro(1-propoxyethyl) group, a perfluoro(1-(2-propoxy-2-methylethoxy) ethyl) group, etc. are preferred.

The group represented by the formula (23) is preferably such that R^{f10} is a C₁₋₈ perfluoroalkyl group, or a C₁₋₈ perfluoroalkyl group having an etheric (ethereal) oxygen atom wherein the number of etheric (ethereal) oxygen atoms is from 1 to 3, and R^{f11} to R^{f14} are each independently a C₁₋₁₂ perfluoroalkyl group (preferably a trifluoromethyl group) or fluorine atom.

As R^{f}, groups represented by the following formulae are particularly preferred.

CF₃CF₂CF₂OCF(CF₃)-

CF₃CF₂OCF₂CF₂OCF₂-

CF₃CF₂OCF₂-

R¹ is a group selected from the groups represented by the above (i) to (vi). In a case where R¹ is a C₁₋₆ alkyl group (group (i)), a methyl group, an ethyl group, a propyl group, an isopropyl group, a butyl group and a hexyl group may, for example, be mentioned.

As examples of a case where R¹ is a monovalent 5-membered ring group containing a hetero atom (group (iii)) or a monovalent 6-membered ring group containing a hetero atom (group (iv)), a 2-thiophenyl group, a 3-thiophenyl group, a 2-pyridyl group, a 3-pyridyl group, a 4-pyridyl group, etc. may be mentioned.

In a case where R¹ is a group (group (v)) having at least one hydrogen atom bonded to a carbon atom in the group selected from the group (i) to group (iv), substituted each independently by a group selected from a hydroxy group, an amino group, a carboxy group and a halogen atom, or a group (group (vi)) having at least one hydrogen atom bonded to a carbon atom in the group selected from the group (i) to group (iv), substituted each independently by a hydroxyl group, an amino group, a carboxy group and a halogen atom, the halogen atom as a substituent in the group (v) and group (vi) may be a fluorine atom, a chlorine atom or an iodine atom, and is preferably a fluorine atom. A C₁₋₆ halogenated alkyl group as a substituent is preferably a C₁₋₆ perfluoroalkyl group.

As examples of the case where R¹ is the group (v) or group (vi), a phenyl group substituted by at least one fluorine atom, a phenyl group substituted by at least one C₁₋₆ perfluoroalkyl group, a phenyl group substituted by at least one fluorine atom and at least one C₁₋₆ perfluoroalkyl group, etc. are preferred.

A preferred example of the group (v) or group (vi) may be a 2-fluorophenyl group, a 4-fluorophenyl group, a 2-trifluoromethylphenyl group, a 4-trifluoromethylphenyl group, a 2-fluoro-4-trifluoromethylphenyl group, a 2,6-difluoro-4-trifluoromethylphenyl group, a 2,4-difluorophenyl group or a 2,4,6-trifluorophenyl group.

Other examples of the group (v) or group (vi) may be 2,6-dichloro-4-trifluoromethylphenyl group, a pyridyl group, a 3-trifluoromethyl-2-pyridyl group, a 4-trifluoromethyl-2-pyridyl group, etc.

The compound (A) of the present invention can be obtained by the following routes by using a compound represented by R^{f}C(O)X as a starting material. However, the production routes of the compound (A) are not limited to the following routes.

### <Root 1: Method for producing compound wherein Q¹ is group (1)>

The compound (A) wherein Q¹ is the group (1) is the following compound (A1). For the compound (A1), a compound (6) represented by the formula R^{f}COX and a carbanion represented by the following formula (7) are reacted to obtain a compound (8), and then, the compound (8) and a compound selected from HC(OR')₃, (CH₃O)₂CHNR"₂ and (CH₃CH₂O)₂CHNR"₂ are reacted to obtain a compound (9). Then, the compound (9) and a compound (5) are reacted to obtain the compound (A1). 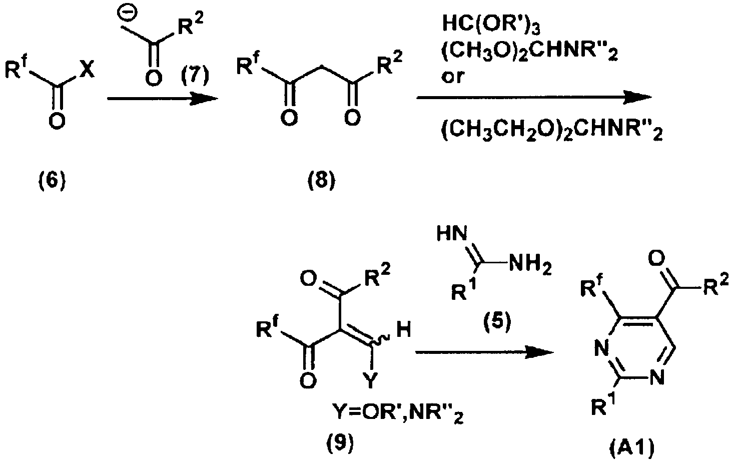
R^{f} and R¹ are as defined above.
X is a halogen atom, a group having a hydrogen atom removed from an N-hydroxysuccinimide group, a C₁₋₆ alkoxy group, a perfluorophenoxy group or a C₁₋₆ fluoroalkoxy group. The halogen atom is preferably a fluorine atom, a chlorine atom, a bromine atom or an iodine atom. The C₁₋₆ alkoxy group is preferably a methoxy group or an ethoxy group. The C₁₋₆ fluoroalkoxy group is preferably a 2-trifluoroethoxy group.
R² is a C₁₋₆ alkyl group, a phenyl group, or a group having at least one hydrogen atom bonded to a carbon atom in a C₁₋₆ alkyl group or a phenyl group, substituted each independently by a group selected from an amino group, a hydroxy group, a halogen atom, a C₁₋₆ alkoxy group, a carboxy group and a trifluoromethyl group.
Y is a group represented by -OR' or -NR"₂, and R' and R" are each independently a C₁₋₃ alkyl group.

The C₁₋₆ alkyl group for R² may, for example, be a methyl group, an ethyl group, a propyl group, an isopropyl group, a butyl group, a hexyl group, etc.

The C₁₋₆ alkoxy group for R² may, for example, be a methoxy group, an ethoxy group, a n-propyloxy group, an isopropyloxy group, a n-butyloxy group, an isobutyloxy group, a t-butyloxy group, etc.

As R², preferred is a phenyl group, a phenyl group substituted by a fluorine atom (e.g. an o-fluorophenyl group, a m-fluorophenyl group, a p-fluorophenyl group, etc.), a phenyl group substituted by a trifluoromethyl group (e.g. an o-trifluoromethylphenyl group, a m-trifluoromethylphenyl group, a p-trifluoromethylphenyl group, etc.), a phenyl group substituted by a hydroxy group (e.g. an o-hydroxyphenyl group, a m-hydroxyphenyl group, a p-hydroxyphenyl group, etc.), a phenyl group substituted by an amino group (e.g. an o-aminophenyl group, a m-aminophenyl group, a p-aminophenyl group, etc.), a phenyl group substituted by a carboxy group (e.g. an o-carboxyphenyl group, a m-carboxyphenyl group, a p-carboxyphenyl group, etc.), etc.

In the process of obtaining a compound (8) by reacting a compound (6) and a carbanion (7), the carbanion (7) can be prepared by letting a compound capable of forming a carbanion (7) be co-existent with an organometallic compound, an alkali metal alkoxide or an alkali metal hydride in a reaction solvent.

The organometallic compound may, for example, be an organoalkali metal compound such as lithium diisopropylamide, lithium hexamethyldisilazide, sodium hexamethyldisilazide or potassium hexamethyldisilazide.

The alkali metal alkoxide may, for example, be sodium ethoxide or potassium (t-butoxide).

The alkali metal hydride may, for example, be sodium hydride.

The reaction solvent to be used in the preparation of the carbanion represented by the formula (7) may, for example, be an ethereal solvent (e.g. tetrahydrofuran, diethyl ether, cyclopentyl methyl ether, 1,4-dioxane, etc.), or a hydrocarbon solvent (e.g. hexane, pentane, benzene, toluene, etc.). As the reaction solvent, one type may be used alone, or two or more types may be used in combination.

For the process of reacting the compound (6) and the compound (7) to obtain the compound (8), conventional techniques and reaction conditions known for similar reactions may be employed.

Next, the compound (8) and HC(OR')₃, (CH₃O)₂CHNR"₂ or (CH₃CH₂O)₂CHNR"₂ are reacted to obtain a compound (9). This reaction can be carried out without a solvent or in an organic solvent.

The organic solvent to be used in the reaction of the compound (8) and HC(OR')₃ may, for example, be acetic acid, acetic anhydride, etc. Further, at the time of the reaction, a Lewis acid such as zinc chloride or tin chloride, may be added. The reaction temperature is preferably from room temperature (25°C) to about 200°C, particularly preferably from 100°C to 160°C.

The organic solvent to be used in the reaction of the compound (8) and (CH₃O)₂CHNR"₂ or (CH₃CH₂O)₂CHNR"₂ may, for example, be an inert solvent such as benzene, toluene, tetrahydrofuran or dioxolane. The reaction temperature is preferably from about 0 to 200°C, more preferably from 50 to 120°C.

Then, the compound (9) and the compound (5) are reacted to obtain the compound (A1). The reaction may be carried out in an organic solvent. As the organic solvent, an inert solvent such as benzene, toluene, methylene chloride, acetonitrile, tetrahydrofuran, dioxolane, N,N-dimethylformamide or N,N-dimethylacetamide, may be mentioned. Among them, toluene or acetonitrile is preferred.

The compound (5) is used in an amount of preferably from 0.5 to 10 times by mol, more preferably from 0.5 to 3 times by mol, to the compound (9). The reaction temperature is preferably from -10°C to 100°C, particularly preferably from 0°C to 40°C. The reaction is preferably carried out in an atmosphere of an inert gas such as nitrogen or argon. The pressure is normally preferably atmospheric pressure or under a slightly pressurized condition of about 0.11 MPa (gauge pressure).

### <Root 2: Method for producing compound wherein Q¹ is group (2)>

The compound (A) wherein Q¹ is the group (2), is the following compound (A2). The compound (A2) can be obtained by reacting a compound (6) represented by the formula R^{f}COX and a compound represented by the following formula (41) to obtain a compound represented by the following formula (10), and then, reacting the compound (10) and a compound represented by the following formula (5). 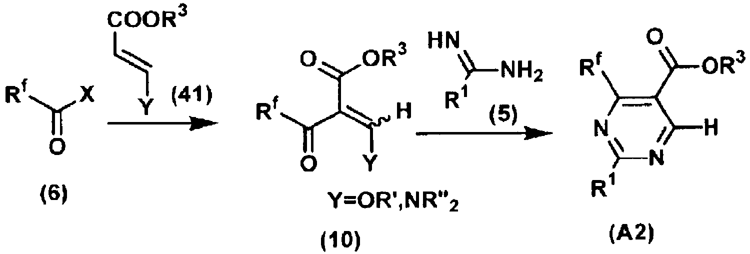
R^{f}, R¹, X and Y are as defined above.
R³ is a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom.

The reaction of the compound (6) represented by R^{f}COX and the compound (41) can be carried out in the presence of a base in an organic solvent.

The organic solvent is preferably an inert solvent, and benzene, toluene, methylene chloride, acetonitrile, tetrahydrofuran, dioxolane, N,N-dimethylformamide or N,N-dimethylacetamide may, for example, be mentioned.

The compound (6) is used in an amount of preferably from 0.5 to 10 times by mol, more preferably from 0.5 to 2 times by mol, to the compound (41). The reaction temperature is preferably from -10°C to 100°C, particularly preferably from 0°C to 40°C.

The above reaction is preferably carried out in an atmosphere of an inert gas such as nitrogen or argon. The pressure is usually from atmospheric pressure to about 0.11 MPa, preferably from atmospheric pressure to a slightly pressurized condition at a level of 0.105 MPa.

As the base, a tertiary amine such as trimethylamine or tributylamine, or a pyridine such as pyridine or 2,6-dimethylpyridine, is preferred, and triethylamine or pyridine is particularly preferred.

In a case where R³ is a C₁₋₆ alkyl group, a methyl group, an ethyl group, a propyl group, an isopropyl group, a butyl group, a hexyl group, etc. may be mentioned. In the case of an aralkyl group, a benzyl group may be mentioned.

The above substituted amino group is a group having one or both of the two hydrogen atoms in an amino group substituted, and as the substituent, a methyl group, an ethyl group, a propyl group, a butyl group, a phenyl group, a benzyl group, a p-methoxybenzyl group, a fluorobenzyl group, etc. may be mentioned, and particularly a methyl group or a benzyl group is preferred.

R³ is preferably a C₁₋₆ alkyl group, an aralkyl group or a substituted aralkyl group, particularly preferably a methyl group, an ethyl group, a benzyl group or a p-methoxybenzyl group.

The compound (41) may, for example, be methyl β-(dimethylamino)acrylate, ethyl β-(diethylamino)acrylate, ethyl β-methoxyacrylate, etc.

The compound (10) obtained by the reaction of the compound (6) and the compound (41), is then reacted with the compound (5). This reaction is preferably carried out in an organic solvent. As the organic solvent, an inert solvent is preferred, and benzene, toluene, methylene chloride, acetonitrile, tetrahydrofuran, dioxolane, N, N-dimethylformamide or N, N-dimethylacetamide may, for example, be mentioned.

The compound (5) is used in an amount of preferably from 0.5 to 10 times by mol, more preferably from 1 to 3 times by mol, to the compound (10). The reaction temperature is preferably from -10°C to 100°C, particularly preferably from 0°C to 40°C. The reaction is preferably carried out in an atmosphere of an inert gas such as nitrogen or argon. The pressure is usually from atmospheric pressure to about 0.11 MPa, preferably from atmospheric pressure to a slightly pressurized condition at a level of 0.105 MPa.

In the reaction of the compound (10) and the compound (5), by changing R¹ in the compound (5), it is possible to obtain a compound (A1) having a desired R¹.

As the compound (5), when R¹ is (i) a C₁₋₆ alkyl group, acetamidine or its hydrochloride (compound wherein R¹ is a methyl group) may be mentioned. Further, when R¹ is (ii) a phenyl group, benzamidine or its hydrochloride (compound wherein R¹ is a phenyl group) may be mentioned. Further, when R¹ is (iii) a monovalent 5-membered ring group containing a hetero atom, 2-thiophene carboimidamide or its hydrochloride (compound wherein R¹ is a 2-thienyl group) may be mentioned. Further, when R¹ is (iv) a monovalent 6-membered ring group containing a hetero atom, 4-pyridine carboximidamide or its hydrochloride salt (compound wherein R¹ is a 4-pyridyl group) may be mentioned. Further, when R¹ is (vi) a group having a hydrogen atom bonded to a carbon atom in a monovalent 6-membered ring containing a hetero atom, substituted by an alkyl halide, 4-trifluoromethyl carboximidamide or its hydrochloride (compound wherein R¹ is a (4-trifluoromethyl) phenyl group) may be mentioned.

When, as the compound (5), a salt such as a hydrochloride, is reacted with the compound (10), an organic base such as triethylamine, tri-isopropylamine or tributylamine may be used. The amount of the organic base is preferably from 1 to 10 times by mol, more preferably from 1 to 2 times by mol, to the compound (5).

### <Root 3: Method for producing compound wherein Q¹ is group (3) to group (6)>

The production route for the compound wherein Q¹ is the group (3) to group (6) can be represented by the following formula. That is, the compound (A3) i.e. the compound (A) wherein Q¹ is the group (3) can be obtained by hydrolyzing the above-mentioned compound (A2). The compound (A3) can be converted to a mixed acid anhydride by using a mixed acid anhydride reagent and then to an azide ketone by a metal azide, and further, while letting it undergo the rearrangement reaction, it is reacted with an alcohol represented by R⁴OH to be converted to a compound (A4) i.e. a compound (A) wherein Q¹ is the group (4). The compound (A4) can be converted by a reaction with an acid, to a compound (A5) i.e. a compound (A) wherein Q¹ is the group (5). The compound (A5) can be converted by a reaction with R⁵C(O)X to a compound (A6) i.e. a compound (A) wherein Q¹ is the group (6).

Here, R⁴ is a C₁₋₆ alkyl group, or a group having at least one hydrogen atom bonded to a carbon atom in a C₁₋₆ alkyl group, substituted by a halogen atom. R⁵ is a C₁₋₆ alkyl group, a phenyl group, an aralkyl group, or a group having at least one hydrogen atom bonded to a carbon atom in a C₁₋₆ alkyl group, a phenyl group or an aralkyl group, substituted each independently by a group selected from an amino group, a hydroxy group, a halogen atom and a trifluoromethyl group.

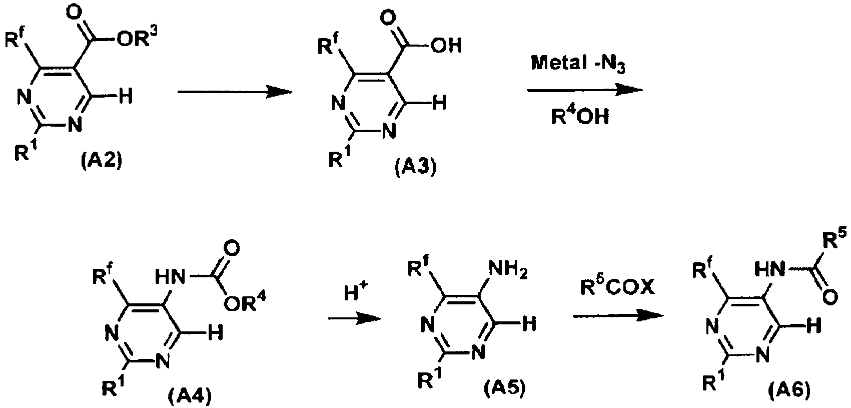

The symbols in the above formula are as defined above.
In the process of obtaining the compound (A3) by hydrolyzing the compound (A2), the hydrolysis can be carried out by heating and refluxing the compound (A2) in a solvent capable of dissolving the compound (A2) (e.g. an alcohol, water, etc.) in the presence of an alkali (e.g., sodium hydroxide, etc.), and then distilling off the solvent, followed further by an extraction operation, etc.

Next, the compound (A3) is converted to a mixed acid anhydride by using a mixed acid anhydride reagent, then reacted with a metal azide (in the formula, a compound represented by Metal-N₃) to form an azide ketone, and further, while letting the rearrangement reaction be taken place, reacted with an alcohol represented by the formula R⁴OH, to obtain a compound (A4) wherein Q¹ is the group (4). R⁴ is preferably a C₁₋₄ alkyl group, or a C₁₋₄ alkyl group substituted by a chlorine atom, and a t-butyl group or a 2,2,2-trichloroethyl group is preferred. The mixed acid anhydride reagent is preferably ethyl chloroformate, methyl chloroformate, etc., particularly preferably ethyl chloroformate. The azide metal compound is preferably sodium azide, etc.

Next, the compound (A4) is reacted with an acid such as hydrochloric acid, sulfuric acid, etc., to obtain a compound (A5) wherein Q¹ is the group (5). The reaction is preferably carried out in a reaction solvent, and the reaction solvent is preferably dioxane, diethyl ether, t-butyl methyl ether, cyclopentyl methyl ether, etc., particularly preferably dioxane or cyclopentyl methyl ether.

Then, by reacting the compound (A5) with a compound represented by the formula R⁵C(O)X (wherein X is particularly a chlorine atom), it is possible to synthesize a compound (A6) wherein Q¹ is the group (6). R⁵ is preferably a phenyl group, a phenyl group substituted by a fluorine atom, a phenyl group substituted by a trifluoromethyl group, or a phenyl group substituted by a fluorine atom and a trifluoromethyl group, particularly preferably a phenyl group, a 4-trifluoromethyl group or a 2-fluoro-4-trifluoromethylphenyl group.

The compound represented by R⁵C(O)X is preferably benzoyl chloride, 2-fluorobenzoyl chloride, 4-trifluoromethylbenzoyl chloride, 2-fluoro-4-trifluoromethylbenzoyl chloride, etc., particularly preferably benzoyl chloride, or 4-trifluoromethylbenzoyl chloride.

The amount of the compound represented by the formula R⁵C(O)X (X=Cl) to the compound (5) is preferably from 1 to 10 times by mol, more preferably from 1 to 3 times by mol. The reaction is preferably carried out in an inert solvent such as methylene chloride, chloroform or toluene by adjusting the reaction temperature to from 0°C to about the refluxing temperature of the solvent, preferably a temperature of from 0 to 40°C, under atmospheric pressure, for a reaction time of from 30 minutes to 48 hours, preferably from 30 minutes to 12 hours.

In a case where a group in a compound of the present invention is substituted by a functional group such as a hydroxy group, a carboxy group or an amino group, protection or deprotection of such a group, may be carried out by employing known methods (e.g. Protective Groups in Organic Synthesis, P.G.M. Wuts & T.W. Greene, Jhon wiley & Sons, Inc., etc.).

Further, for post-treatment techniques after the reaction, common operations in organic syntheses can be employed.

In an isolation operation, as the case requires, activated carbon treatment, distillation, recrystallization, crystallization, column chromatography, etc. may be carried out.

Among the compounds (A) of the present invention, particularly preferred compounds are shown below.

In the following formulae, Et means an ethyl group, and tBu means a t-butyl group.

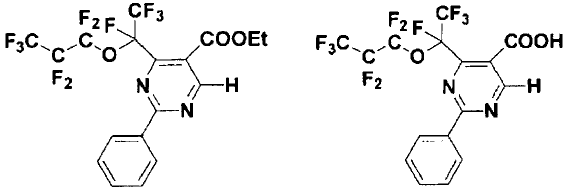

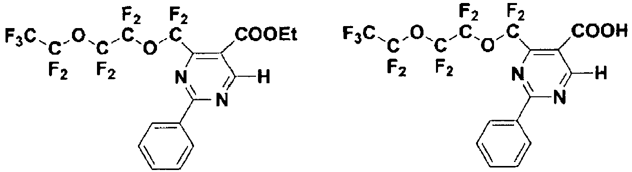

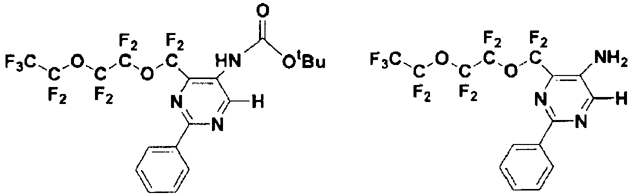

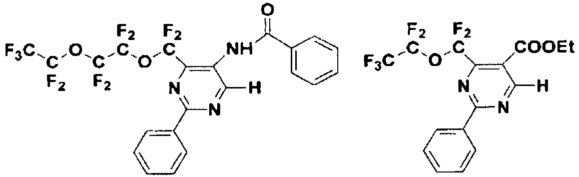

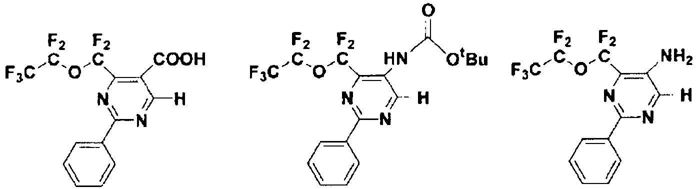

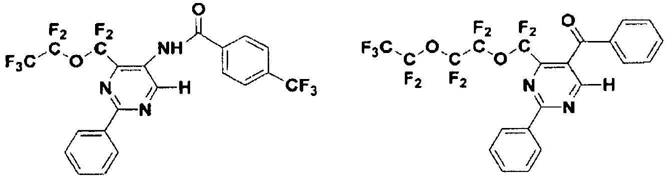

According to the present invention, a novel pyrimidine derivative is provided wherein on three carbon atoms out of the four carbon atoms in a pyrimidine ring, a fluorinated alkyl group having an etheric (ethereal) oxygen atom, and two specific substituents, are respectively bonded, and on the remaining one carbon atom, a hydrogen atom is bonded.

The perfluoroalkyl group having an etheric (ethereal) oxygen atom in the compound is a group capable of being bended and further is hydrophobic. Accordingly, when used as a pharmaceutical or agricultural chemical, the compound of the present invention has ester bonds, amide bonds, or carboxy groups, present in substituents represented by Q¹, and thus is capable of bonding to active sites of amino acids constituting a protein in vivo, and further capable of expressing physiological activities by hydrophobic interaction with biological molecules, by perfluoroalkyl groups having hydrophobicity, whereby it is capable of expressing high pharmacological activities as a pharmaceutical or agricultural chemical.

When the compound of the present invention is to be used as an agricultural chemical, here, the agricultural chemical means pesticides on plants, and, for example, means a herbicide, a plant fungicide, an insecticide, etc. Specifically, it shows powerful antibacterial activities, for example, against pathogenic bacteria against rice, wheat, barley, etc., particularly against rice blast fungus (Pyricularia oryzae), rice Botrytis cinerea (Botrytis cinerea), etc.. Thus, formulations containing compounds of the present invention are useful as plant fungicides.

When the compound of the present invention is to be used as a herbicide, at least one type of the compound of the present invention is incorporated in the formulation usually in a proportion of from 0.1 to 99 mass%, preferably from 1 to 60 mass%. The method of use may vary depending upon the purpose of use, the target plant, the duration of use, etc., but soil treatment or foliar application is usually appropriate. The concentration for use may vary depending upon the purpose of use, the target plant, the duration of use, etc., but the amount of the active ingredient to be applied is usually in a range of from about 1 to 50 g per one are.

The compound of the invention may be mixed with various carriers depending upon the use situation and may be formulated into, for example, granules, a wettable powder, an emulsion, etc. The carriers mentioned here may be solid or liquid, or a combination thereof. For example, a solid carrier such as clay, talc, diatomaceous earth, bentonite, etc., or a liquid carrier such as water, an alcohol, acetone, benzene, toluene, xylene, solvent naphtha, cyclohexane, etc. may be used. Further, an emulsifier, a stabilizer, a dispersing agent, a suspending agent, a spreading agent, a penetrating agent, a wetting agent, etc. to be used for formulations of agricultural chemicals, may be added.

When the compound of the present invention is to be used as a plant fungicide, such a formulation contains at least one type of the compound of the present invention usually in a proportion of from 0.1 to 99 mass%, more preferably from 1 to 60 mass%. Such a formulation may be used alone, or as diluted. The concentration in use may vary depending upon the intended purpose, the use object and the target plant, but is usually in a range of from 1 to 50,000 ppm, preferably from about 100 to 5,000 ppm. The amount of the active ingredient to be applied is usually from 1.0 g to 5 kg, preferably from about 2 g to 100 g per hectare.

When used as a plant fungicide, it can be used in the form of a customary formulation, such as a solution, a wettable powder, an emulsion, a suspension, a liquid concentrate, tablets, granules, an aerosol, a powder, a paste or a smoking agent. Such a formulation may be obtained by a conventional method of mixing at least one type of the compound of the present invention with a suitable solid or liquid carrier, and, as the case requires, suitable auxiliary agents for improvement of dispersibility or other properties of the active substance (e.g. a surfactant, a spreading agent, a dispersing agent, a stabilizer, etc.).

The formulation containing the compound of the present invention may be used as mixed with other fungicides, insecticides, herbicides or fertilizers, etc. in addition to the above components.

Further, ester bonds, amide bonds or carboxy groups present in substituents represented as Q¹, can be converted to optional functional groups by their conversion reactions, whereby various pyrimidine compounds may be derivatized. Accordingly, the compound of the present invention is also useful as a novel intermediate.

### EXAMPLES

Now, the present invention will be described with reference with Examples, but the present invention is not limited to these Examples. In the formulae in Examples, C₃F₇O- is CF₃CF₂CF₂O-, Et means an ethyl group, and tBu means a t-butyl group.

The nuclear magnetic resonance spectrum (NMR) was measured by using JNM-AL300 manufactured by JEOL Ltd.

Here, ¹H-NMR represents the chemical shift δ of signal (unit: ppm) (splitting patterns, integral) using tetramethylsilane as the internal standard. Symbol "s" means singlet, "d" means doublet, "t" means triplet, "q" means quartet, "m" means multiplet, "br" means broad, "J" means a coupling constant, and "CDCl₃" means deuterated chloroform.

Further, ¹⁹F-NMR represents the chemical shift δ of signal (unit: ppm) (integral of fluorine atoms) using CCl₃F as the standard.

### <Synthesis Example 1>

In a nitrogen atmosphere, ethyl β-dimethylamino acrylate (1.43 g) was dissolved in toluene (10 ml), and pyridine (0.8 g) was added at room temperature. Here, perfluoro(2-methyl-3-oxa-hexanoyl) fluoride (3.2 g) was dropwise added, followed by stirring for 10 hours at the same temperature. To the reaction mixture, water (20 ml) was added, and the organic phase was separated. Further, the aqueous phase was extracted with toluene (10 ml), and the extract was combined with the previous organic phase. This organic phase was dried over anhydrous magnesium sulfate, and the solvent was distilled off under reduced pressure, to obtain a compound represented by the following formula (4.5 g).

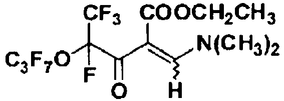

¹H-NMR (300 MHz, CDCl₃)δ=1.31 (t, J=7.2 Hz, 3H), 3.07(s, 3H), 3.31 (s, 3H), 4.17(q, J=7.2 Hz, 2H), 7.67(s, 1 H).

### <Example 1: Production Example of Compound (A2-1)>

The compound (0.91 g, 2.0 mmol) obtained in Synthesis Example 1 was dissolved in acetonitrile (4 ml), and in a nitrogen atmosphere at room temperature, benzamidine hydrochloride (0.94 g, 6.0 mmol) and triethylamine (1 g, 12.0 mmol) were added, followed by stirring at 105°C for 1 hour. The solvent was distilled off under reduced pressure, followed by purification by silica gel column chromatography (hexane:ethyl acetate = 10:1 (volume ratio, the same applies hereinafter)) to obtain a compound (0.77 g) represented by the following formula (A2-1).

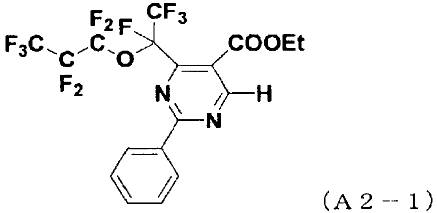

¹H-NMR (300 MHz, CDCl₃)δ=1.39(t, J=7. Hz, 3H), 4.46(q, J=7.2 Hz, 2H), 7.5-7.6(m, 3H), 8.5-8.6(m, 2H), 9.06(s, 1 H).
¹⁹F-NMR (300 MHz, CDCl₃, CCl₃F standard)δ=-81.5(1F), -81.7(6F), -84.3(1 F), -129.7(1F), -143.3(2F).

### <Example 2: Production Example of Compound (A3-1)>

The compound (A2-1) (0.71 g, 1.4 mmol) obtained in Example 1 was dissolved in ethanol (3 ml), and a sodium hydroxide (60 mg, 1.8 mmol) aqueous solution (3 ml) was added at room temperature, followed by heating and refluxing at 105°C. After 1 hour, the solvent was distilled off under reduced pressure; water (5 ml) was added; the pH was adjusted to about 2 by 10 mass% sulfuric acid; and the precipitated white solid was collected by filtration, to obtain a compound (0.53 g) represented by the following formula (A3-1).

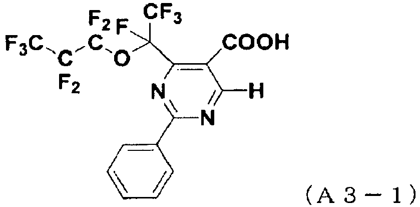

¹H-NMR (300 MHz, CDCl₃)δ=6.0-6.3(m, 1 H), 7.5-7.7(m, 3H), 8.5-8.6(m, 2H), 9.21 (s, 1 H).
¹⁹F-NMR (300 MHz, CDCl₃, CCl₃F standard)δ=-80.2(1F), -81.3(3F), -81.4(3F), -84.3(1 F), -128.9(1F), -130.2(2F).

### <Synthesis Example 2>

Except that perfluoro(2-methyl-3-oxahexanoyl) fluoride was changed to perfluoro(3,6-dioxaoctanoyl) fluoride (1.74 g), the reaction was conducted under the same conditions as in Synthesis Example 1 for 2 hours to obtain the following compound (2.58 g).

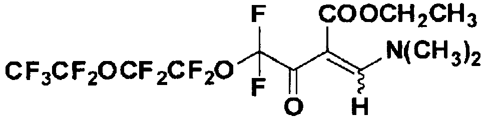

¹H-NMR (300 MHz, CDCl₃)δ=1.29(t, J=7.2 Hz, 3H), 2.87(s, 3H), 3.31(s, 3H), 4.15(q, J=7.2 Hz, 2H), 7.70(s, 1 H).
¹⁹F-NMR(300 MHz, CDCl₃, CCl₃F standard)δ=-74.2 to -74.3(2F), -86.2 to -86.3(3F), -87.6 to -88.1(6F).

### <Example 3: Production Example of Compound (A2-2)>

The compound (0.47 g, 1.0 mmol) obtained in Synthesis Example 2 was dissolved in acetonitrile (4 ml), and in a nitrogen atmosphere at room temperature, benzamidine hydrochloride (0.47 g, 3.0 mmol) and triethylamine (0.51 g, 6.0 mmol) were added, followed by stirring at 105°C for 1 hour. The solvent was distilled off under reduced pressure, followed by purification by silica gel column chromatography (hexane:ethyl acetate = 10:1) to obtain a compound (0.43 g) represented by the following formula (A2-2).

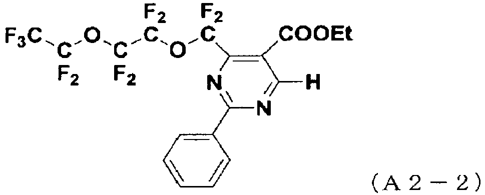

¹H-NMR (300 MHz, CDCl₃)δ=1.41(t, J=7.2 Hz, 3H), 4.46(q, J=7.2 Hz, 2H), 7.4-7.7(m, 3H), 8.5-8.6(m, 2H), 9.28(s, 1 H).
¹⁹F-NMR (300 MHz, CDCl₃, CCl₃F standard)δ=-69.9 to -70.0(2F), -87.0 to -87.1 (3F), -87.1 to -87.2(2F), -99.8 to -88.9(4F).

### <Example 4: Production Example of Compound (A3-2)>

The compound (A2-2) (0.43 g, 0.81 mmol) obtained in Example 3 was dissolved in ethanol (2 ml), and an aqueous solution (2 ml) of sodium hydroxide (40 mg, 1.0 mmol) was added at room temperature, followed by heating and refluxing at 105°C. After 1 hour, the solvent was distilled off under reduced pressure; water (5 ml) was added; the pH was adjusted to about 2 with 10 mass% sulfuric acid; and the precipitated white solid was collected by filtration, to obtain a compound (0.30 g) represented by the following formula (A3-2).

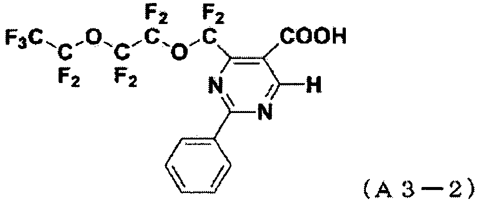

¹H-NMR (300 MHz, CDCl₃)δ=2.7-3.0(m, 1H), 7.5-7.7(m, 3H), 8.0-8.1(m, 2H), 9.38(s, 1 H).
¹⁹F-NMR (300 MHz, CDCl₃, CCl₃F standard)δ=-70.2 to -70.3(2F), -87.1 (3F), -88.7 to -88.8(2F), -88.8 to -89.0(4F).

### <Example 5: Production Example of Compound (A4-1)>

The compound (A3-2) (2.71 g) obtained in Example 4 was dissolved in acetone (30 ml), and triethylamine (0.84 g) and ethyl chloroformate (0.71 g) were added at 0°C, followed by stirring for 20 minutes. Then, at the same temperature, an aqueous solution (3 ml) of sodium azide (0.75 g) was added to the above reaction mixture, followed by stirring for 1 hour. Water (200 ml) was added to this reaction mixture; the organic phase was separated; and further, the aqueous phase was extracted with toluene (30 ml). The organic phases were combined and dried over anhydrous magnesium sulfate, and low boiling point compounds were distilled off under reduced pressure to obtain a solution of about 5 ml.

Then, a mixture of t-butyl alcohol (10 ml) and toluene (10 ml) was heated and refluxed, and here, the above reaction mixture was dropwise added, followed by a reaction for 2 hours. Low boiling point compounds were distilled off from the reaction mixture under reduced pressure, and the residue was purified by silica gel column chromatography (hexane:ethyl acetate = 20:1) to obtain a compound (2.0 g) represented by the following formula (A4-1).

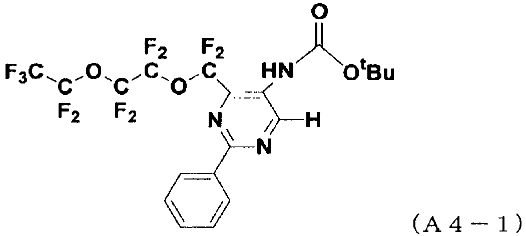

¹H-NMR (300 MHz, CDCl₃)δ=1.52(s, 9H), 6.76(brs, 1H)7.4-7.5(m, 3H), 8.4-8.5(m, 2H), 9.70(s, 1 H).
¹⁹F-NMR (300 MHz, CDCl₃, CCl₃F standard)δ=-69.8(2F), -87.0(3F), -88.5(2F), -88.9(4F).

### <Example 6: Production Example of Compound (A5-1)>

The compound (A4-1) (0.92 g) obtained in Example 5 was dissolved in dioxane (5 ml) and 2M hydrochloric acid (5 ml), followed by heating and refluxing for 5.5 hours. After neutralizing with a 5 mass% sodium hydroxide solution, the reaction mixture was extracted with chloroform (5 ml), and then, low boiling point compounds were distilled off under reduced pressure. The residue was purified by silica gel column chromatography (hexane:ethyl acetate = 5:1) to obtain a compound (0.40 g) represented by the following formula (A5-1).

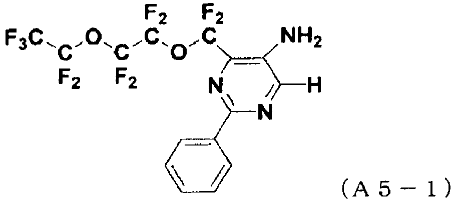

¹H-NMR (300 MHz, CDCl₃)δ=4.28(brs, 2H), 7.3-7.6(m, 3H), 8.3-8.5(m, 2H), 8.45(s, 1 H). ¹⁹F-NMR (300 MHz, CDCl₃, CCl₃F standard)δ=-71.4(2F), -87.1 (3F), -88.6(2F), -88.9(4F).

### <Example 7: Production Example of Compound (A6-1)>

The compound (A5-1) (0.10 g) obtained in Example 6 was dissolved in pyridine (2 ml), and at room temperature, benzoyl chloride (0.058 g) was added, followed by stirring for 20 hours. The reaction mixture was concentrated and purified by silica gel column chromatography (hexane:ethyl acetate = 10:1) to obtain a compound (0.14 g) represented by the following formula (A6-1).

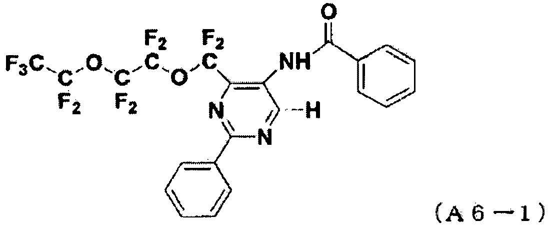

¹H-NMR (300 MHz, CDCl₃)δ=7.0-7.2(m, 3H), 7.7-7.7(m, 2H), 7.7-7.9(m, 2H), 8.2-8.3(m, 2H), 8.4-8.5(m, 2H), 9.94(s, 1 H).
¹⁹F-NMR (300 MHz, CDCl₃, CCl₃F standard)δ=-69.4(2F), -86.9(3F), -87.7(2F), -88.2(4F).

### <Synthesis Example 3>

Except that the perfluoro(2-methyl-3-oxa-hexanoyl) fluoride was changed to perfluoro(3-oxapentanoyl) fluoride (7.0 g), the reaction was conducted under the same conditions as in Synthesis Example 1 for 18 hours, to obtain the following compound (13.5 g).

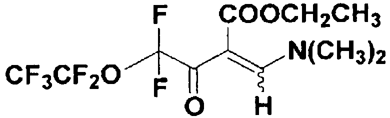

¹H-NMR (300 MHz, CDCl₃)δ=1.29(t, J=7.2 Hz, 3H), 2.88(brs, 3H), 3.33(s, 3H), 4.21 (q, J=7.2 Hz, 2H), 7.69(s, 1 H).
¹⁹F-NMR (300 MHz, CDCl₃, CCl₃F standard)δ=-75.0(2F), -87.1 (3F), -88.5(2F).

### <Example 8: Production Example of Compound (A2-3)>

The compound (2.84 g, 8.0 mmol) obtained in Synthesis Example 3 was dissolved in acetonitrile (15 ml), and in a nitrogen atmosphere at room temperature, benzamidine hydrochloride (3.76 g, 24.0 mmol) and triethylamine (4.04 g, 40.0 mmol) were added, followed by stirring at 105°C for 1 hour. The solvent was distilled off under reduced pressure, followed by purification by silica gel column chromatography (hexane:ethyl acetate = 10:1) to obtain a compound (3.28 g) represented by the following formula (A2-3).

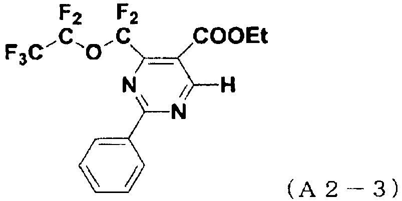

¹H-NMR (300 MHz, CDCl₃)δ=1.42(t, J=7.2 Hz, 3H), 4.45(q, J=7.2 Hz, 2H), 7.0-7.2(m, 3H), 8.5-8.6(m, 2H), 9.25(s, 1 H).
¹⁹F-NMR (300 MHz, CDCl₃. CCl₃F standard)δ=-70.1(2F), -87.1 (3F), -87.6(2F).

### <Example 9: Production Example of Compound (A3-3)>

The compound (A2-3) (3.0 g, 7.0 mmol) obtained in Example 8 was dissolved in ethanol (5 ml), and an aqueous solution (5 ml) of sodium hydroxide (0.36 g, 9.0 mmol) was added at room temperature, followed by heating and refluxing at 105°C. After 45 minutes, the solvent was distilled off under reduced pressure; water (10 ml) was added; the pH was adjusted to about 2 with 10 mass% sulfuric acid; and the precipitated white solid was collected by filtration, to obtain a compound (2.0 g) represented by the following formula (A3-3).

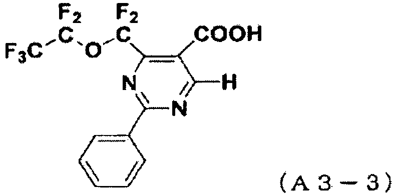

¹H-NMR (300 MHz, CDCl₃)δ=7.5-7.6(m, 3H), 8.5-8.6(m, 2H), 9.36(s, 1H). ¹⁹F-NMR (300 MHz, CDCl₃, CCl₃F standard)δ=-70.3(2F), -87.1 (3F), -87.8(2F).

### <Example 10: Production Example of Compound (A4-2)>

The compound (A3-3) (1.75 g) obtained in Example 9 was dissolved in acetone (25 ml), and triethylamine (0.68 g) and ethyl chloroformate (0.57 g) were added at 0°C, followed by stirring for 20 minutes. Then, at the same temperature, an aqueous solution (3 ml) of sodium azide (0.61 g) was added to the above reaction mixture, followed by stirring for 1 hour. Water (160 ml) was added to this reaction mixture, the organic phase was separated, and further, the aqueous phase was extracted with toluene (25 ml). The organic phases were combined and dried over anhydrous magnesium sulfate, and low boiling point compounds were distilled off under reduced pressure to obtain a solution of about 5 ml.

Next, a mixture of t-butyl alcohol (9 ml) and toluene (9 ml) was heated and refluxed, and here, the above reaction mixture was dropwise added, followed by a reaction for 2 hours. Low boiling point compounds were distilled off from the reaction mixture under reduced pressure, and the residue was purified by silica gel column chromatography (hexane:ethyl acetate = 20:1) to obtain a compound (1.7 g) represented by the following formula (A4-2).

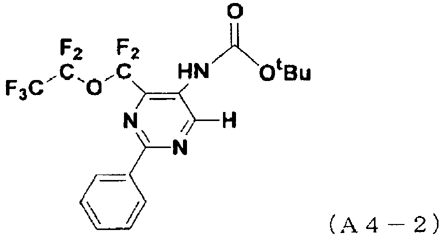

¹H-NMR (300 MHz, CDCl₃)δ=1.55(s, 9H), 6.91 (brs, 1 H)7.4-7.5(m, 3H), 8.4-8.5(m, 2H), 9.66(s, 1H).
¹⁹F-NMR (300 MHz, CDCl₃, CCl₃F standard)δ=-70.3(2F), -87.0(3F), -88.3(2F).

### <EXAMPLE 11: Production Example of Compound (A5-2)>

A compound (0.38 g) represented by the following formula (A5-2) was obtained under the same conditions as in Example 6, except that the compound (A4-1) was changed to the compound (A4-2) (1.7 g).

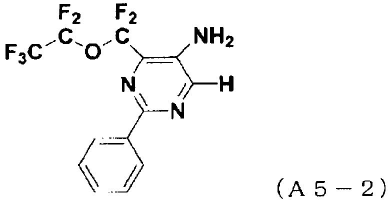

¹H-NMR(300 MHz, CDCl₃)δ=4.39(brs, 2H), 7.4-7.5(m, 3H), 8.3-8.4(m, 2H), 8.46(s, 1H). ¹⁹F-NMR(300 MHz, CDCl₃, CCl₃F standard)δ=-71.4(2F), -87.0(3F), -88.5(2F).

### <Example 12: Production Example of Compound (A6-2)>

The compound (A5-2) (0.38 g) obtained in Example 11 was dissolved in methylene chloride (3 ml), and at room temperature, 4-dimethylaminopyridine (0.30 g) and p-trifluoromethylbenzoyl chloride (0.46 g) were added, followed by stirring for 15 hours. A saturated sodium hydrogen carbonate aqueous solution (20 ml) was added to the reaction mixture, followed by extraction with methylene chloride (10 ml). The organic layer was concentrated under reduced pressure and purified by silica gel column chromatography (hexane:ethyl acetate = 4:1) to obtain a compound (0.23 g) represented by the following formula (A6-2).

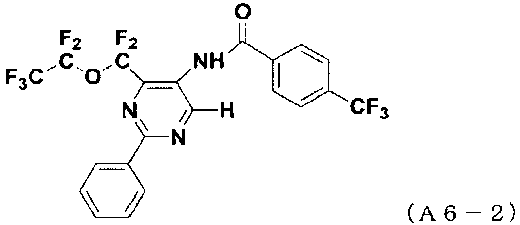

¹H-NMR (300 MHz, CDCl₃)δ=7.5-7.6(m, 3H), 7.83(d, J=8.1 Hz, 2H), 7.99(d, J=8.1 Hz, 2H), 8.16(s, 1 H), 8.4-8.5(m, 2H), 9.92(s, 1 H).
¹⁹F-NMR (300 MHz, CDCl₃, CCl₃F standard)δ=-63.6(3F), -69.7(2F), -86.9(3F), -88.3(2F).

### <Synthesis Example 4>

Acetophenone (2.4 g) was dissolved in tetrahydrofuran (40 ml), and t-butoxy potassium (5.0 g) was added at 0°C. After stirring at room temperature for 15 minutes, perfluoro(3,6-dioxaoctanoic acid) methyl ester (9.0 g) was added, followed by stirring for 18 hours. Water (10 ml) was added to the reaction mixture, followed by neutralization with 10 mass% sulfuric acid. The organic phase was separated, the aqueous phase was extracted with ethyl acetate (10 ml), and the organic phases were combined and dried over anhydrous sodium sulfate. After distilling off low boiling point compounds under reduced pressure, purification was conducted by column chromatography (hexane-ethyl acetate = 10/1) to obtain the following compound (8.1 g).

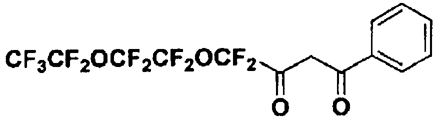

¹H-NMR (300 MHz, CDCl₃)δ=6.57(s, 2H), 7.4-7.5(m, 2H), 7.5-7.6(m, 1H), 7.8-7.9(m, 2H).
¹⁹F-NMR (300 MHz, CDCl₃, CCl₃F standard)δ=-79.5(2F), -87.1 (3F), -88.5 to -88.8(2F), 89.0-89.2(4F).

### <Example 13: Production Example of Compound (A1-1)>

The compound (0.67 g) obtained in Synthesis Example 4 was dissolved in acetic anhydride (2 ml), and triethyl orthoformate (0.28 g) was added, followed by stirring at 140°C for 3 hours. Low-boiling point compounds were distilled off under reduced pressure, and the residue was washed with ethanol (2 ml), and further low-boiling point compounds were distilled off under reduced pressure. To this reaction mixture, acetonitrile (3 ml) was added, and benzamidine hydrochloride (0.39 g) was added, followed by stirring at room temperature for 20 hours. Low-boiling point compounds were distilled off under reduced pressure, and the residue was purified by silica gel column chromatography (hexane-ethyl acetate = 10/1), to obtain a compound (0.06 g) represented by the following formula (A1-1).

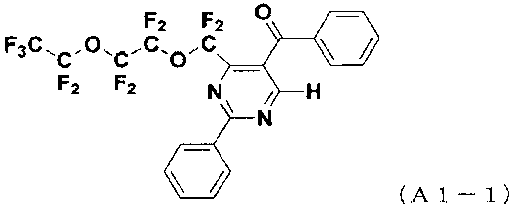

¹H-NMR (300 MHz, CDCl₃)δ=7.4-7.8(m, 7H), 7.9-8.0(m, 3H), 9.69(s, 1 H). ¹⁹F-NMR (300 MHz, CDCl₃, CCl₃F standard)δ=-74.7(2F), -87.0(3F), -88.3(2F), -88.6(4F).

### <Synthesis Example 5>

The following compound (2.0 g) was obtained by conducting the reaction for 3 hours under the same conditions as in Synthesis Example 1, except that the ethyl β-dimethylamino acrylate was changed to trans-4-(dimethylamino)-3-buten-2-one (0.56 g), and the perfluoro(2-methyl-3-oxa-hexanoyl) fluoride was changed to perfluoro(3,6-dioxaoctanoyl) fluoride (1.74 g).

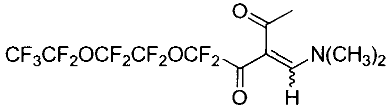

¹H-NMR (300 MHz, CDCl₃)δ=2.42(s, 3H), 2.83(brs, 3H), 3.34(brs, 3H), 7.63(s, 1 H). ¹⁹F-NMR (300 MHz, CDCl₃, CCl₃F standard)δ=-73.5(2F), -87.0(3F), -88.6(2F), -88.7(4F).

### <Example 14: Production Example of Compound (A1-1)>

The compound (0.30 g, 0.73 mmol) obtained in Synthesis Example 5 was dissolved in acetonitrile (6 ml), and in a nitrogen atmosphere at room temperature, benzamidine hydrochloride (0.34 g, 2.2 mmol) and triethylamine (0.37 g, 3.6 mmol) were added, followed by stirring at 105°C for 0.5 hour. The solvent was distilled off under reduced pressure, followed by purification by silica gel column chromatography (hexane:ethyl acetate = 15:1 to 10:1) to obtain a compound (0.23 g) represented by the following formula (A1-1).

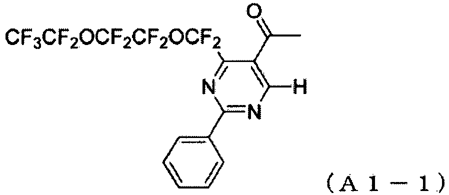

¹H-NMR (300 MHz, CDCl₃)δ=2.63(s, 3H), 7.5-7.7(m, 3H), 8.5-8.6(m, 2H), 9.00(s, 1H). ¹⁹F-NMR (300 MHz, CDCl₃, CCl₃F standard)δ=-68.9(2F), -87.0(3F), -88.6(2F), -88.7(4F).

### <Example 15: Antimicrobial activity tests against rice Botrytis cinerea and rice blast>

Test specimen (compound (A2-1) and compound (A5-1)) and reference agents i.e. iprodione and captan, were, respectively, dissolved in dimethyl sulfoxide (DMSO) and adjusted to 10,000 ppm. To a flat bottom 96 well microplate, 2 µ/ml of each test solution was dispensed, and then, 198 µ/ml of conidia of rice Botrytis cinerea (Botrytis cinerea AARF-033) cultured on a potato-glucose agar medium, and suspended in a potato-glucose broth and adjusted to 1×10⁴ conidia/ml, were dispensed, followed by stirring by a micro mixer. At that time, the concentration of each test specimen and reference agent, was 100 ppm. Thereafter, culturing under standing-still condition was conducted at 25°C for 4 days, whereby the antimicrobial activities were evaluated.

Similarly, with respect to conidia of rice blast fungus (Magnaporthe grisea) cultured in an oatmeal medium, a suspension was prepared, and by using benomyl and fluoxastrobin as reference agents, the antimicrobial activities were evaluated. At that time, the concentration of each test specimen and reference agent, was 100 ppm.

The results of each antibacterial activity evaluation are shown in Table 1.

**[Table 1]**

| Test specimens and reference agents | Botrytis cinerea AARF-033 | Magnaporthe grisea |
|---|---|---|
| A2-1 | At least 95% inhibition | At least 95% inhibition |
| A5-1 | At least 95% inhibition | At least 95% inhibition |
| Iprodione | At least 95% inhibition | |
| Captan | At least 95% inhibition | |
| Benomyl | | At least 95% inhibition |
| Fluoxastrobin | | At least 95% inhibition |

From the results in Table 1, each of compound (A2-1) and compound (A5-1) was confirmed to have antibacterial activities comparable to the reference agents, against rice Botrytis cinerea and rice blast fungus.

### INDUSTRIAL APPLICABILITY

The compound of the invention is a novel pyrimidine derivative capable of exhibiting high pharmacological activities, as a pharmaceutical or agricultural chemical, and is useful also as a novel intermediate that can be derivatized into various pyrimidine compounds.

The entire disclosure of Japanese Patent Application No. 2014-100655 filed on May 14, 2014 including specification, claims and summary is incorporated herein by reference in its entirety.

## Claims

1. A compound represented by the following formula (A): 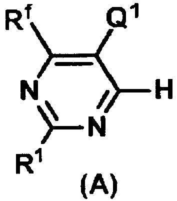 wherein
R^{f}, R¹ and Q¹ have the following meanings:
R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
Q¹: (1) a group represented by R²C(O)- (wherein R² is a C₁₋₆ alkyl group, a phenyl group, or a group having at least one hydrogen atom bonded to a carbon atom in a C₁₋₆ alkyl group or a phenyl group, substituted each independently by a group selected from an amino group, a hydroxy group, a halogen atom, a C₁₋₆ alkoxy group, a carboxy group and a trifluoromethyl group), (2) a group represented by R³OC(O)- (wherein R³ is a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom), (3) a carboxy group, (4) a group represented by R⁴OC(O)NH- (wherein R⁴ is a C₁₋₆ alkyl group, or a group having at least one hydrogen atom bonded to a carbon atom in a C₁₋₆ alkyl group, substituted by a halogen atom), (5) an amino group, or (6) a group represented by R⁵C(O)NH- (wherein R⁵ is a C₁₋₆ alkyl group, a phenyl group, an aralkyl group, or a group having at least one hydrogen atom bonded to a carbon atom in a C₁₋₆ alkyl group, a phenyl group or an aralkyl group, substituted each independently by a group selected from an amino group, a hydroxy group, a halogen atom and a trifluoromethyl group.).

2. The compound according to Claim 1, wherein R^{f} in the formula (A) is a group represented by (R^{f10})(R^{f11})(R^{f12})C-O-(R^{f13})(R^{f14})C- (wherein R^{f10} to R^{f14} are each independently a C₁₋₁₂ perfluoroalkyl group, a C₁₋₁₂ perfluoroalkyl group having an etheric (ethereal) oxygen atom at least between carbon-carbon atoms or at a bond terminal, or a fluorine atom, and the total of the number of carbon atoms and the number of oxygen atoms is from 3 to 20.).

3. The compound according to Claim 1 or 2, wherein the compound represented by the formula (A) is a compound represented by the following formula (A1): 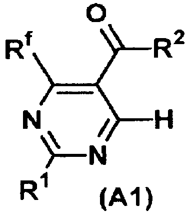 wherein R^{f}, R¹ and R² are as defined above.

4. The compound according to Claim 1 or 2, wherein the compound represented by the formula (A) is a compound represented by the following formula (A2): 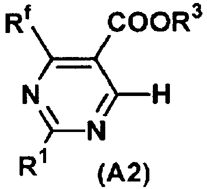 wherein R^{f}, R¹ and R³ are as defined above.

5. The compound according to Claim 1 or 2, wherein the compound represented by the formula (A) is a compound represented by the following formula (A3): 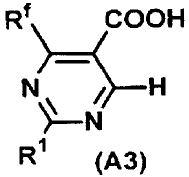 wherein R^{f} and R¹ are as defined above.

6. The compound according to Claim 1 or 2, wherein the compound represented by the formula (A) is a compound represented by the following formula (A5): 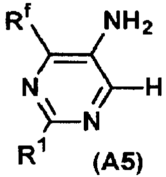 wherein R^{f} and R¹ are as defined above.

7. The compound according to Claim 1 or 2, wherein the compound represented by the formula (A) is a compound represented by the following formula (A6): 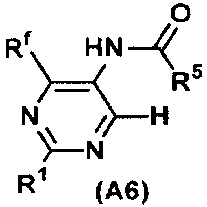 wherein R^{f}, R¹ and R⁵ are as defined above.

8. The compound according to any one of Claims 1 to 7, wherein R¹ is a phenyl group, or a group having at least one hydrogen atom bonded to a carbon atom in a phenyl group, substituted each independently by a group selected from a hydroxy group, an amino group, a carboxy group and a halogen atom.

9. A method for producing a compound represented by the following formula (A1), which comprises reacting a compound represented by the following formula (9) and a compound represented by the following formula (5): 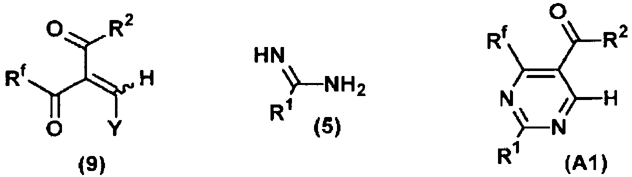 wherein
R^{f}, Y, R¹ and R² have the following meanings:
R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
Y: a group represented by -OR' or -NR"₂, wherein R' and R" are each independently a C₁₋₆ alkyl group,
R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
R²: a C₁₋₆ alkyl group, a phenyl group, or a group having at least one hydrogen atom bonded to a carbon atom in a C₁₋₆ alkyl group or a phenyl group, substituted each independently by a group selected from an amino group, a hydroxy group, a halogen atom, a C₁₋₆ alkoxy group, a carboxy group and a trifluoromethyl group.

10. A method for producing a compound represented by the following formula (A2), which comprises reacting a compound represented by the following formula (10) and a compound represented by the following formula (5): 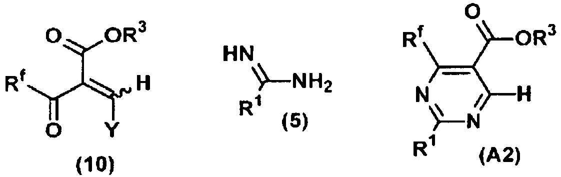 wherein
R^{f}, Y, R¹ and R³ have the following meanings:
R^{f} : a group having an etheric (ethereal) oxygen atom inserted between carbon-carbon atoms in a C₂₋₁₉ perfluoroalkyl group, wherein the total of the number of carbon atoms and the number of oxygen atoms is from 3 to 20,
Y: a group represented by -OR' or -NR"₂, wherein R' and R" are each independently a C₁₋₃ alkyl group,
R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
R³: a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom.

11. A method for producing a compound represented by the following formula (A6), which comprises hydrolyzing a compound represented by the following formula (A2) under a basic condition, to obtain a compound represented by the following formula (A3); converting the compound represented by the formula (A3) to a mixed acid anhydride, and then to an azide ketone by an metal azide compound; while further letting it undergo the rearrangement reaction, reacting it with a compound represented by the formula R⁴-OH (wherein R⁴ is a C₁₋₆ alkyl group, or a group having at least one hydrogen atom bonded to a carbon atom in a C₁₋₆ alkyl group, substituted by a halogen atom) to obtain a compound represented by the following formula (A4), then reacting the compound represented by formula (A4) with an acid, to obtain a compound represented by the following formula (A5), and then reacting the compound represented by the formula (A5) with a compound represented by the formula R⁵COX (wherein R⁵ is a C₁₋₆ alkyl group, a phenyl group, an aralkyl group, or a group having at least one hydrogen atom bonded to a carbon atom in a C₁₋₆ alkyl group, a phenyl group or an aralkyl group, substituted each independently by a group selected from an amino group, a hydroxy group, a halogen atom and a trifluoromethyl group, and X is a halogen atom, a group obtained by removing a hydrogen atom from N-hydroxysuccinimide, a C₁₋₆ alkoxy group, a perfluorophenoxy group or a C₁₋₆ fluoroalkoxy group.). 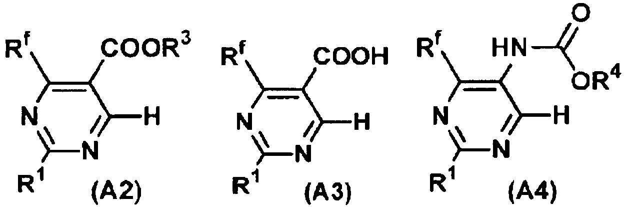 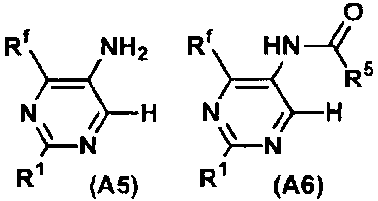 wherein
R^{f}, R¹ and R³ have the following meanings, and R⁴ and R⁶ are as defined above:
R^{f} : a group having an etheric oxygen atom inserted between carbon-carbon atoms in a C₂₋₁₉ perfluoroalkyl group, wherein the total of the number of carbon atoms and the number of oxygen atoms is from 3 to 20,
R¹: (i) a C₁₋₆ alkyl group, (ii) a phenyl group, (iii) a monovalent 5-membered ring group containing a hetero atom, (iv) a monovalent 6-membered ring group containing a hetero atom, (v) a group having at least one hydrogen atom bonded to a carbon atom in a group selected from the above group (i) to group (iv), substituted each independently by a group selected from a hydroxy group, an amino group, a carboxy group and a halogen atom, or (vi) a group having at least one hydrogen atom bonded to a carbon atom in a group selected from the above group (ii) to group (iv), substituted each independently by a C₁₋₆ halogenated alkyl group,
R³: a C₁₋₆ alkyl group, an aralkyl group, a phenyl group, or a group having at least one hydrogen atom in a phenyl group, substituted each independently by a group selected from an amino group, a substituted amino group, a C₁₋₆ alkoxy group and a halogen atom.

12. An agricultural chemical containing a compound selected from the compound as defined in any one of Claims 1 to 8 and pharmacologically effective salts of said compound.
